# EUROPÄISCHE PATENTSCHRIFT

(11) **EP 1 713 756 B1**
(45) Veröffentlichungstag und Bekanntmachung des Hinweises auf die Patenterteilung: **19.08.2015**
(21) Anmeldenummer: 05701305.4
(22) Anmeldetag: 02.02.2005
(51) Int. Cl.: C07C 201/08, C07C 205/06, C07C 201/14

(54) **VERFAHREN ZUR HERSTELLUNG VON DINITROTOLUOL**
METHOD FOR PRODUCING DINITROTOLUENE
PROCEDE DE PRODUCTION DE DINITROTOLUENE

(30) Priorität: 05.02.2004 DE 102004005913
(43) Veröffentlichungstag der Anmeldung: 25.10.2006
(73) Patentinhaber: BASF SE, 67056 Ludwigshafen (DE)
(72) Erfinder: BÜTTNER, Johannes, 01945 Ruhland (DE); MACKENROTH, Wolfgang, 67089 Bad Dürkheim (DE); HERMANN, Heinrich, 50858 Köln (DE); KONIECZNY, Peter, 14513 Teltow (DE); GEBAUER, Jürgen, 53840 Troisdorf (DE)
(86) Internationale Anmeldenummer: PCT/EP2005/001017
(87) Internationale Veröffentlichungsnummer: WO 2005/075407

(56) Entgegenhaltungen:
- US-A- 4 367 347
- US-A- 4 663 490
- US-A- 5 679 873
- US-A1- 2002 091 290

## Beschreibung

Die Erfindung betrifft ein Verfahren zur Herstellung von Dinitrotoluol (DNT) durch Nitrierung von Toluol mit Salpetersäure in Gegenwart von Schwefelsäure in zwei Stufen im Gegenstrom.

Dinitrotoluol (DNT) ist ein Zwischenprodukt für die Herstellung von Toluoldiisocyanat (TDI), neben Methylendiisocyanat (MDI) das wichtigste Vorprodukt zur Herstellung von Polyurethanen.

Die Nitrierung von Toluol zum DNT erfolgt, wie beispielsweise in H. Hermann, J. Gebauer, P. Konieczny, Industrial Nitration of Toluene to Dinitrotoluene in ASC-Symphosium, series 623, 234-249, 1996 ed. L.F. Albright, R.V.C. Carr, R.J. Schmitt beschrieben, vorwiegend isotherm mit Salpetersäure in Gegenwart von Schwefelsäure (Mischsäure) als Katalysator in zwei Stufen kontinuierlich im Gegenstrom in einem Zwei-Phasen-System derart, dass auf der Stufe der Nitrierung des Toluol zu Mononitrotoluol (MNT) (MNT-Stufe) die aus der DNT-Stufe anfallende Abfallsäure mit der Salpetersäure aufgestärkt, mit dem Toluol gemischt, nach Umsetzung des Toluol zu MNT die Phasen getrennt werden und das MNT in einer zweiten Stufe (DNT-Stufe)mit Salpetersäure in Gegenwart von Schwefelsäure zu DNT umgesetzt wird.

Die Abfallsäure aus der Nitrierung der ersten Stufe mit einem Schwefelsäuregehalt von mindestens 70 bis 71 %, einem Restgehalt von Salpetersäure von 0,1 bis 0,5 %, einem Gehalt an Nitrose von 0,4 bis 1,5 Gew.-%, angegeben als salpetrige Säure (HNO₂); noch gelöstem MNT von ca. 0,2 bis 0,45 % und einem Wassergehalt von maximal 26,6 bis 29,3 % wird einer Reinigung vor der Weiterverwendung, wie Rückführung in den Nitrierprozess nach Aufkonzentrierung, zugeführt.

Das Roh-MNT, das neben Spuren an Toluol im Bereich von 0,1 bis 0,3 Gew.-%, noch Salpetersäure, Stickstoffdioxid (NO₂), Nitrokresole und das in der Abfallsäure aus der Nitrierung der zweiten Stufe noch gelöste DNT enthält, wird in die DNT-Stufe eingeführt und dort vollständig mit einem frischen Gemisch aus Salpetersäure und Schwefelsäure so zu DNT umgesetzt, dass ein DNT entsprechend der gewünschten Spezifikation, wie MNT-Gehalt < 0,1 %, TNT-Gehalt < 500 ppm, Ortho-Isomere max. 4,5 %, erhalten wird. Nach Trennung der Phasen wird das Roh-DNT so gewaschen, dass die im DNT gelöste Salpeter- und Schwefelsäure größtenteils zurückgewonnen und in die Nitrierung auf der MNT-Stufe eingespeist werden kann (s. EP 0 736 514).

Die Abfallsäure aus der DNT-Stufe mit einer Schwefelsäurekonzentration von ca. 78,0 bis 79,0 Prozent, Salpetersäure von ca. 1 bis 1,5 % und Nitrose aus der Oxidation der Nitrokresole im MNT von ca. 0,8 -1,5 % wird nach Aufstärken mit frischer Salpetersäure in der MNT-Stufe als Mischsäure zur Nitrierung eingesetzt.

Als Schwefelsäure für die Nitrierung des MNT zu DNT wird üblicherweise eine Schwefelsäure mit einem Gehalt von etwa 96 % eingesetzt, die entweder frisch hergestellt oder durch Aufkonzentrieren der Abfallsäure aus der MNT-Stufe in einer Rekonzentrieranlage gewonnen wurde.

Neben diesem Standardverfahren der 2-stufigen kontinuierlichen isothermen Nitrierung wurde in EP 903 336 auch vorgeschlagen die Nitrierung von Toluol zu DNT mit Mischsäure in 3 Stufen kontinuierlich durchzuführen oder adiabatisch einstufig oder zweistufig so durchzuführen, dass, wie in EP 597 361 und EP 696 570 beschrieben, die gesamte Reaktionswärme aus der Nitrierung des Toluol zu DNT oder nur aus der DNT-Stufe, wie in EP 696 571 beschrieben, zum Abtrennen des Reaktionswassers aus der Nitrierung und des durch die Salpetersäure in die Abfallsäure eingebrachten Wassers ausgenutzt wird. Außerdem wurde in US 5 948 944 und US 2 362 743 vorgeschlagen, die Nitrierung von Toluol zu DNT nur in Salpetersäure als Reaktionsmedium durchzuführen und so den Einsatz von Schwefelsäure zu vermeiden.

Bei allen Verfahren zur Herstellung von DNT durch Nitrierung von Toluol ist für eine wirtschaftliche Verfahrensführung Voraussetzung, dass das Reaktionsmedium, z.B. Schwefelsäure oder Salpetersäure, so wieder aufbereitet wird, dass es in den Nitrierprozess erneut als Reaktionsmedium eingesetzt werden kann, wie beispielsweise in EP 155 586 und US-Pat. 5,275,701 beschrieben.

Wesentlich für die Auswahl eines Nitrierverfahrens ist, auch aus Gründen der Umweltbelastung, dass die Verluste an Säuren, sowohl Schwefel- als auch Salpetersäure, durch Nebenreaktionen so gering wie möglich sind.

Dieses Ziel wird üblicherweise einmal dadurch erreicht, dass der Abfallschwefelsäure aus der Nitrierung zum MNT nach einer Entfernung der noch gelösten Nitroaromaten, deren Gehalt je nach Verhältnis MNT/DNT in der Abfallsäure und der Konzentration an Schwefelsäure in der Abfallsäure 0,15 bis 0,45 Gew.-% beträgt, der Salpetersäure und der Nitrose in einem ein- bis mehrstufigen Prozess das Wasser bis zu einer Schwefelsäurekonzentration entzogen wird, die es gestattet, diese aufkonzentrierte Schwefelsäure als Frischsäure mit einer Konzentration von 88 bis 94 % Schwefelsäure oder nach einer weiteren Aufkonzentrierung in einer Hochkonzentrierstufe auf 94 bis 98 % Schwefelsäure in die DNT-Nitrierstufe zurückzuführen, wie beispielsweise in EP 155 586 beschrieben.

Zur Minimierung der Verluste an Salpetersäure, die nicht in das Endprodukt umgewandelt wird, wird, wie beispielsweise in EP 0 736 514, beschrieben, die Salpetersäure aus der Wäsche des Roh-DNT, als schwache Säure mit einem Gesamtsäuregehalt von 23,73 bis 40 % Gesamtsäure zusammen mit der Salpetersäure aus der Abgaswäsche und der Strippung der Abfallsäure direkt oder nach Aufkonzentrierung in die Nitrierung zurückgeführt. Dadurch wird neben der Verbesserung der Ausbeute an DNT bezogen auf den Salpetersäureeinsatz auf bis über 98 % gleichzeitig die Belastung des Abwassers an Nitrat wesentlich reduziert.

Bei Einsatz von teilkonzentrierter Schwefelsäure aus der Nitrierung von Toluol zu MNT für die Herstellung von DNT ist es nicht nötig, alle Verunreinigungen aus der Nitrierung, vollständig aus der teilkonzentrierten Schwefelsäure vor Rückführung in die Nitrierung zu entfernen.

Dadurch ist es möglich, auch mit geringen Aufbereitungsgraden z.B. einer Schwefelsäure von 88 bis 94 %, die noch Spuren an DNT und Nitrose enthält, zu arbeiten, ohne dass die Produktqualität negativ beeinflusst wird. Dadurch können die Kosten für die Schwefelsäureaufkonzentrierung niedrig gehalten werden, da die aufwendige Hochkonzentrierung der Schwefelsäure entfallen kann.

Neben einer geschlossenen Kreisführung der Schwefelsäure im Prozess mit einem minimalen Verbrauch an Schwefelsäure und einem Umsatz der eingesetzten Salpetersäure von mehr als 98 % zum Produkt sind für eine moderne Anlage zur kontinuierlichen isothermen Herstellung von DNT aus Toluol in zwei Stufen im Gegenstrom zusätzlich weitere aufwendige technische Maßnahmen nötig, um eine "kontrollierte Nitrierung" durchführen zu können.

So gilt es, während der Nitrierung undefinierte Betriebszustände zu verhindern und damit auszuschließen, dass es zu einer "unkontrollierten" Nitrierung - verbunden mit hoher Wärmeentwicklung - kommt, was im Extremfall zur Explosion führen kann. Dieser Forderungskatalog gilt vor allem dann, wenn das hochreaktive Toluol noch im Reaktionsgemisch vorliegt.

### Dies wird üblicherweise dadurch erreicht, dass

a) in zwei definierten Stufen derart im Gegenstrom gearbeitet wird, dass in der MNT-Stufe nur Toluol zu MNT umgesetzt wird mit einer Nitriersäure und unter Bedingungen, bei denen eine unkontrollierte weitere Umsetzung des gebildeten MNT bei der vorgegebenen Verweilzeit zum DNT weitgehend ausgeschlossen ist und auf der DNT-Stufe das MNT vollständig zum DNT entsprechend der Spezifikation umgesetzt wird.
b) in allen Nitrierstufen nur in der Abfallsäure bei möglichst niedriger Schwefelsäure- und Salpetersäurekonzentration nitriert wird,
c) das zweiphasige Nitriergemisch stets als homogene Emulsion im Reaktor vorliegt,
d) die Reaktionswärme aus der Nitrierung und aus dem Zumischen von konzentrierten Mischsäuren zum Reaktionsgemisch effektiv abgeführt wird, was nur möglich ist, wenn neben ausreichender Kühlfläche bei Zugabe von zu nitrierendem Produkt wie Toluol und MNT das Nitriergemisch als Emulsion vorliegt,
e) der Anteil an noch nicht umgesetztem Produkt (Toluol, MNT) im Reaktionsgemisch in den einzelnen Reaktoren möglichst niedrig ist,
f) eine Phasentrennung der Nitriergemische in Organik- und Säurephase in Gegenwart überschüssiger Salpetersäure nur erfolgt, wenn in der Organikphase kein bzw. nur noch geringe Mengen zu nitrierendes Produkt vorhanden sind,
g) eine Phasentrennung des Nitriergemisches mit noch nicht vollständig umgesetztem Produkt (Toluol, MNT) nur erfolgt, wenn der Salpetersäuregehalt in der Nitriersäure 0 bzw. so gering ist, dass bei einer unkontrollierten Nitrierung die freigesetzte Wärme nicht zu undefinierten Betriebszuständen wie Zersetzung, Ausgasung führen kann.

Dieser Forderungskatalog gilt vor allem dann, wenn noch unumgesetztes Toluol im Reaktionsgemisch vorliegt.

Eine kontrollierte Nitrierung, die diese sicherheitstechnischen Forderungen erfüllt, ist gegeben, wenn eine Phasentrennung des Nitriergemisches nur durchgeführt wird, wenn kein zu nitrierendes Produkt mehr in der entsprechenden Organphase auf der jeweiligen Nitrierstufe vorliegt (Toluol in der MNT-Stufe und MNT in der DNT-Stufe) und der Salpetersäuregehalt möglichst niedrig ist.

Bei den bekannten technischen Verfahren erfolgt daher auf der MNT-Stufe die Phasentrennung üblicherweise dann, wenn das Toluol zu mehr als 99,5 % umgesetzt ist, beschrieben beispielsweise in EP 903 336, US 3,092,671 und EP 066 202 entsprechend einem Toluolgehalt von ca. 0,1 bis 0,5 %, siehe beispielsweise s. H. Hermann, J. Gebauer, P. Konieczny, Industrial Nitration of Toluene to Dinitrotoluene in ASC-Symphosium, series 623, 234-249,1996 ed. L.F. Albright, R.V.C. Carr, R.J. Schmitt, Tab. II,).

Der Salpetersäuregehalt in der MNT-Abfallsäure liegt dann üblicherweise bei ca. 0,4 bis 1,0 % Salpetersäure. Unter diesen Bedingungen ist eine unkontrollierte Nitrierung mangels Toluol in der Organphase nicht mehr möglich. Die gesamte Reaktionswärme, die aus der Umsetzung des Toluol zu MNT anfällt, wurde bei den Bedingungen einer kontrollierten Nitrierung definiert abgeführt.

Umgekehrt wird, wenn im MNT auf der MNT-Stufe noch ein merklicher Gehalt von Toluol, beispielsweise von 3,5 bis 5 % im MNT vorliegt, die Phasentrennung erst durchgeführt, wenn in der Abfallsäure zum Zeitpunkt der Phasentrennung keine Salpetersäure mehr vorliegt, sondern diese vor der Phasentrennung vollständig verbraucht wurde, siehe US 2,947,791.

Neben diesen kontinuierlichen technischen Verfahren mit definierten Bedingungen pro Verfahrensstufe wurde in PL 126 069 vorgeschlagen, auf der MNT-Stufe bereits ein MNT-/DNT-Gemisch herzustellen und erst dann die Phasentrennung durchzuführen, um das verbliebene MNT im MNT/DNT-Gemisch in der DNT-Stufe vollständig umzusetzen

Bei diesen Bedingungen mit dem wesentlich weniger reaktiven MNT im Gemisch mit DNT und in Gegenwart einer Abfallsäure mit einer Schwefelsäurekonzentration, die nur eine langsame Umsetzung von MNT zu DNT erlaubt, ist die Gefahr von unkontrollierten Reaktionen wesentlich reduziert.

Zur Erzielung eines möglichst vollständigen selektiven Umsatzes des Toluols auf der MNT-Stufe wird bei den bekannten technischen Verfahren die Schwefelsäurekonzentration in der Nitriersäure und die Reaktionstemperatur so gewählt, dass nur MNT gebildet wird, aber eine weitere Reaktion zu DNT gar nicht bzw. möglichst langsam erfolgt.

Diese zweistufige Verfahrensweise mit selektivem Umsatz des Toluols zu MNT bei einer Reaktionstemperatur von vorzugsweise 35 bis 45°C, einer Abfallsäure mit vorzugsweise 70 bis 72 % Schwefelsäure und einem Salpetersäuregehalt von vorzugsweise 0,3 bis 0,7 % in der MNT-Stufe und in der DNT-Stufe bei einer Reaktionstemperatur von vorzugsweise 60 bis 70°C, einer Abfallsäure mit vorzugsweise 80 bis 82 % Schwefelsäure (ohne das gelöste DNT in der DNT-Abfallsäure) und einem Salpetersäuregehalt von vorzugsweise 1,0 bis 1,5 % in der DNT-Stufe bietet den Vorteil, dass die Zugabe der Reaktionspartner Toluol und Salpetersäure auf der MNT-Stufe bzw. Salpetersäure und Schwefelsäure auf der DNT-Stufe stöchiometrisch durch eine einfache Massendosierung erfolgen kann und eine aufwendige analytische Kontrolle des Reaktionsgemisches während der Nitrierung auf den einzelnen Stufen entfallen kann, wie sie erforderlich wird, wenn eine Mischnitrierung zu MNT/DNT-Gemischen in der MNT-Stufe mit Schwefelsäurekonzentrationen in der MNT-Abfallsäure erfolgt, mit denen in Gegenwart von nicht umgesetztem Toluol bereits ein merklicher Umsatz des MNT zu DNT möglich ist.

Für diese selektive vollständige Umsetzung des Toluols in der MNT-Stufe bzw. des MNT in der DNT-Stufe bei der kontinuierlichen isothermen Nitrierung des Toluols zu DNT in zwei Stufen im Gegenstrom wird üblicherweise die Nitrierung in Rührkesselkaskaden auf den einzelnen Stufen durchgeführt. So wurde in US 3,434,802, EP 903 336, EP 066 202, PL 126 089 und US 2,947,791 vorgeschlagen, die technische Nitrierung des Toluols zu MNT in einer zwei- bis vierstufigen Rührkesselkaskade durchzuführen.) und die Umsetzung des MNT zu DNT gleichfalls in einer zwei- bis vierstufigen Rührkesselkaskade durchzuführen.

Bei dieser Arbeitsweise wird in Verbindung mit den entsprechenden Nitriersäuren nicht nur ein Umsatz des Toluols in der MNT-Stufe von mehr als 99,5 % erzielt, sondern auch ein DNT, das die geforderten Spezifikationen an Reinheit (MNT <0,1 %, TNT <0,1 %) bei technisch praktikablen Verweilzeiten des Nitriergemisches in den einzelnen im Gegenstrom geführten kontinuierlichen Gleichstrom-Nitrierstufen einhält.

Aufgabe der Erfindung war es, das Verfahren zur Herstellung von DNT insbesondere bezüglich der Anzahl der verwendeten Apparate in Verbindung mit einer Verringerung des Reaktionsvolumens weiter zu vereinfachen und insbesondere die Zahl der eingesetzten Reaktoren zu vermindern.

Es hat sich nun überraschenderweise gezeigt, dass es möglich ist, die isotherme Nitrierung von Toluol zu DNT abweichend von den Nitrierbedingungen des Standes der Technik so durchzuführen, dass auf der MNT-Stufe zum Zeitpunkt der Phasentrennung im MNT noch ein erhöhter Toluolgehalt und gleichzeitig auch in der Nitriersäure noch ein Salpetersäuregehalt vorliegt, ohne dass undefinierte Betriebszustände mit entsprechenden Sicherheitsrisiken auftreten, sofern die Phasentrennung des MNT von der Abfallsäure so erfolgt, dass eine Weiterreaktion des Toluols mit der Salpetersäure zuverlässig verhindert wird.

Gegenstand der Erfindung ist somit ein Verfahren zur Herstellung von Dinitrotoluol, umfassend die Schritte
a) Umsetzung von Toluol mit Salpetersäure in Anwesenheit von Schwefelsäure zu Mononitrotoluol
b) Trennung des Reaktionsprodukts aus Schritt a) in eine Mononitrotoluol enthaltende organische Phase und eine Schwefelsäure enthaltende wässrige Phase,
c) Umsetzung der Mononitrotoluol enthaltenden organischen Phase mit Salpetersäure in Anwesenheit von Schwefelsäure zu Dinitrotoluol,
d) Trennung des Reaktionsprodukts aus Schritt c) in eine Dinitrotoluol enthaltende organische Phase und eine Schwefelsäure enthaltende wässrige Phase,
dadurch gekennzeichnet, dass das Reaktionsprodukt aus Schritt a) einen Gehalt an Toluol von 3.5 - 8 Gew.-%, vorzugsweise 3,5 bis 5 Gew.-%, bezogen auf die organische Phase, und einen Gehalt an Salpetersäure von 0,1 bis 1,2 Gew.-%, bezogen auf die wässrige Phase, aufweist und die Phasentrennung in Schritt b) so erfolgt, dass eine Weiterreaktion des Toluols mit der Salpetersäure verhindert wird.

Die Weiterreaktion kann durch eine schnelle und effektive Trennung der organischen und der anorganischen Phase verhindert werden. Eine solche Trennung wird mittels dynamischer Separatoren durchgeführt.

Die organische Phase aus Schritt b) kann hierbei ohne weitere Aufarbeitung in Schritt c) überführt werden.

Die wässrigen Phasen aus den Schritten b) und d) können, gegebenenfalls nach einer Aufarbeitung und Aufkonzentration, wieder in Schritt a) und Schritt c) eingesetzt werden. Die Aufkonzentration erfolgt üblicherweise durch Abtrennung des Wassers, insbesondere mittels Destillation. Die Schwefelsäure hat nach der Aufkonzentration vorzugsweise eine Konzentration im Bereich zwischen 85 und 96 %.

Die Temperatur der Stufe a) liegt vorzugsweise im Bereich zwischen 35 und 70°C, vorzugsweise zwischen 45 und 55°C.

Das Molverhältnis von Salpetersäure zu Toluol liegt im Schritt a) vorzugsweise im Bereich zwischen 0,95 und 1,12. Das Molverhältnis von Salpetersäure zu MNT in Schritt c) liegt vorzugsweise zwischen 1,03 und 1,10.

Die Temperatur bei der Stufe c) liegt zumeist im Bereich zwischen 60 und 85°C, vorzugsweise im Bereich zwischen 65 und 80°C.

Die Konzentration der in den Stufen a) und c) eingesetzten Salpetersäure liegt üblicherweise zwischen 58 und 100 %. In der Praxis ist es üblich, entweder mit einer Salpetersäurekonzentration im Bereich zwischen 58 und 68 % oder mit einer Salpetersäurekonzentration im Bereich zwischen 95 und 99,9 % zu arbeiten.

Ein MNT mit den erfindungsgemäßen Restgehalten von Toluol und Salpetersäure zum Zeitpunkt der Phasentrennung kann mit der üblichen Anzahl von 2 bis 4 Rührkesseln bei sonst gleichen Nitrierbedingungen erhalten werden, wenn bei niedrigen Nitriertemperaturen gearbeitet wird, wie z.B. in US Pat. 3 708 546 beschrieben, oder die Nitrierung bei einer Schwefelsäurekonzentration von 62 bis 64 % in der Abfallsäure durchgeführt wird.

Beide Vorgehensweisen sind für eine großtechnische Herstellung von DNT wenig geeignet.

Eine weitere Möglichkeit zur Erreichung der erfindungsgemäßen Gehalte an Toluol und Salpetersäure besteht darin, entgegen der üblichen Verfahrensweise die Nitrierung des Toluols zu MNT nicht in einer mehrstufigen Rührkesselkaskade durchzuführen, sondern nur in einem Rührkessel, und dann die Phasentrennung durchzuführen. Bei sonst gleichen Reaktionsbedingungen wie in der mehrstufigen Rührkesselkaskade wird durch die Verkürzung der Verweilzeit um ca. 50 % das Toluol nicht vollständig umgesetzt.

Durch Variation der Nitriertemperatur in Stufe a), beispielsweise. eine Temperaturerhöhung im Reaktor auf 55°C, kann der Toluolgehalt im MNT und der Salpetersäuregehalt in der Nitriersäure gezielt eingestellt werden.

Üblicherweise wurde im Stand der Technik bei der zweistufigen Nitrierung des Toluol zu DNT im Gegenstrom nach dem Standardverfahren auf der MNT-Stufe mindestens zwei Reaktoren eingesetzt, wobei z.B. das Toluol, Salpetersäure und die DNT-Endsäure entweder nur in einen Reaktor der Stufe a) eingespeist werden und der andere Reaktor als Verweilzeitbehälter dient, oder die Salpetersäure wird zusammen mit einem Teil des Toluols in einem Reaktor der Stufe a) eingespeist und der Rest des Toluols in den zweiten Reaktor der Stufe a), um das Isomerenverhältnis 2.4-, 2.6-DNT zu optimieren.

Es zeigte sich nun überraschenderweise, dass es möglich ist, ca. 85 bis 95 % des eingesetzten Toluols im ersten Reaktor der MNT-Stufe umzusetzen. Ein solches MNT steht im Gleichgewicht mit einer Nitriersäure von ca. 72 bis 73 % Schwefelsäure und einem Salpetersäuregehalt von max. 1,2 % bei einer Nitriertemperatur von 55°C.

Wird in der Stufe a) nur mit einem Reaktor gearbeitet und die Phasentrennung des Nitriergemisches direkt nach dem Reaktor derart durchgeführt, dass keine unkontrollierte Weiterreaktion erfolgen kann und wird das so erhaltene MNT mit bis zu 8 Gew. %

Toluol direkt in die DNT-Stufe eingespeist, zeigt sich überraschenderweise, dass im Vergleich zu dem Standardverfahren, bei dem mit einem MNT nach der Phasentrennung mit einem Toluolgehalt < 0,2 % gearbeitet wird, auch ein MNT mit einem Resttoluolgehalt von bis zu. 8 Gew. % zu einem DNT umgesetzt werden kann, das alle Spezifikationsparameter erfüllt. Auch die Bildung von Nebenprodukten wie Nitrocresole und Nitrose verändert sich nicht.

Eine Anpassung der Dosierung an Salpetersäure auf der MNT-Stufe und an Schwefelsäure und Salpetersäure auf der DNT-Stufe entsprechend den geänderten Umsätzen auf den einzelnen Stufen ist überraschenderweise nicht nötig.

Dieses unerwartete Verhalten zeigt das hohe Puffervermögen der Nitriergemische gegen geringfügige Dosierschwankungen an Toluol, Salpetersäure und Schwefelsäure. Der nicht vollständige Umsatz des Toluols von bis zu 15 % der eingesetzten Menge auf der MNT-Stufe macht es nicht nötig, die sicherheitstechnisch optimalen Dosierverhältnisse für die einzelnen Stufen zu ändern.

Es hat sich zusätzlich gezeigt, dass an Stelle einer drei- bis vierstufigen Reaktor-, insbesondere Rührkesselkaskade für die DNT-Stufe der vollständige Umsatz eines MNT mit einem Toluolgehalt von bis zu 8 Gew. % auch in einer Reaktor-, insbesondere Rührkesselkaskade, bestehend aus maximal zwei Reaktoren bei reduzierter Verweilzeit einwandfrei möglich ist. Auch hier kann zusätzlich durch Variation der Nitriertemperatur, beispielsweise ein Erhöhen der Temperatur im ersten und zweiten oder auch nur im zweiten Reaktor auf bis zu 85°C der Rest-MNT-Gehalt im DNT auf <0,1 % eingestellt werden. Ein zusätzlicher Vorteil des Arbeitens mit nur zwei Reaktoren auf der DNT-Stufe ist, das der Gehalt an Trinitrotoluol (TNT) im DNT signifikant abnimmt und sogar unter die Nachweisgrenze abfallen kann.

Der leicht erhöhte Restgehalt an Salpetersäure im Vergleich zu einer vollständigen Umsetzung des Toluols in zwei Reaktoren von ca. 1,2 % in der Abfallsäure aus der MNT-Stufe wird bei Wiederaufbereitung der Abfallschwefelsäure zum Zwecke der Rückführung in die Nitrierung ebenfalls zurückgewonnen und in die Nitrierung zurückgeführt.

Die Erfindung soll an den nachfolgenden Beispielen näher erläutert werden.

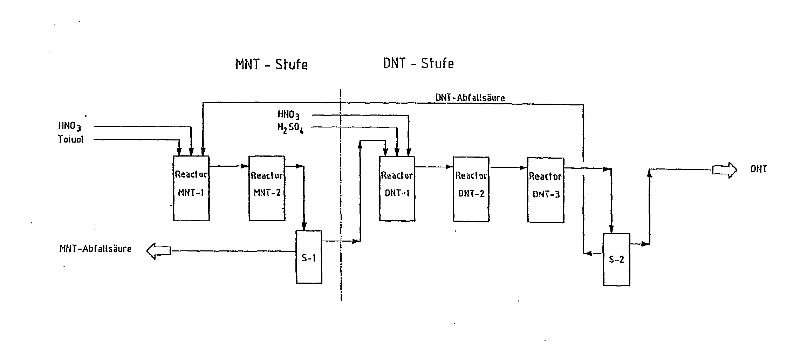

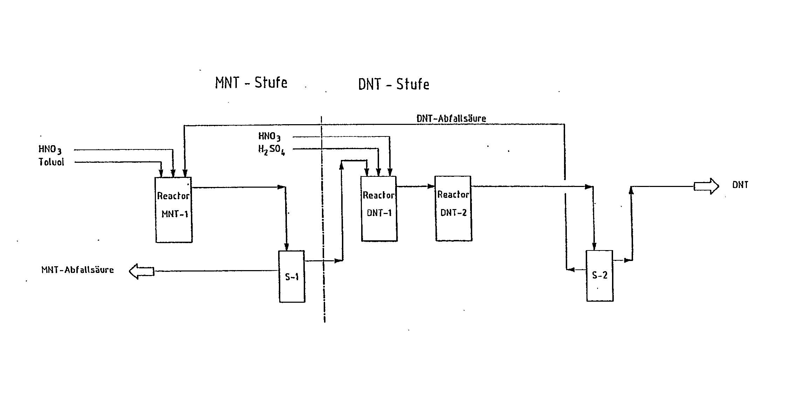

Allgemeine Nitrierbedingungen für Beispiel 1 (Vergleichsbeispiel, Abb. 1) und Beispiel 2 (erfindungsgemäßes Beispiel, Abb. 2):

Bei Beispiel 1 wurde in der MNT-Stufe mit 2 Rührkesseln und in der DNT-Stufe mit drei Rührkesseln gearbeitet. In Beispiel 2 wurde in der MNT-Stufe mit einem Rührkessel und in der DNT-Stufe mit zwei Rührkesseln gearbeitet.

870 kg Schwefelsäure 94,5 % aus der Wiederaufbereitung einer MNT-Abfallsäure wurden zusammen mit 372 kg Salpetersäure 99,7 % und dem MNT aus der MNT-Stufe nach Phasentrennung im Scheider S1 in den Reaktor DNT-1 der DNT-Stufe eingespeist.

Nach Durchlauf des Reaktionsgemisches durch die Rührkesselkaskade der DNT-Stufe (DNT-1 bis DNT-n), wurde das Gemisch DNT/DNT-Abfallsäure im Scheider S2 getrennt.

Das DNT (1000 kg) wurde in einer nachfolgenden Wäsche von allen sauren Verunreinigungen (Salpetersäure, Schwefelsäure, Nitrose) und den Nitrokresolen befreit.

Die Abfallsäure wurde in die MNT-Stufe im Reaktor MNT-1 mit 519 kg Toluol und 341 kg Salpetersäure 99,7 % zusammen mit der zurückgewonnenen Salpetersäure aus der Wäsche des DNT und der Wiederaufbereitung der MNT Abfallsäure eingespeist.

Nach Durchlauf des Reaktionsgemisches durch die Rührkesselkaskade der MNT-Stufe (MNT-1 bis MNT-n) wurde das Gemisch MNT/MNT-Abfallsäure im Scheider S1 getrennt. Das Roh-MNT wurde wie vorstehend beschrieben in die DNT-Stufe eingespeist. Die MNT-Abfallsäure wurde von den gelösten Nitroaromaten, der Rest-Salpetersäure und der Nitrose befreit und nach Aufkonzentrieren zu Schwefelsäure 94,5 % in den Prozess zurückgeführt.

In Tabelle 1 sind die Zusammensetzungen der Abfallsäure und der Produkte für eine Nitrierung nach dem Standardverfahren (2 Reaktoren auf der MNT-Stufe, 3 Reaktoren auf der DNT-Stufe, Abb. 1) und für das erfindungsgemäße, Verfahren (1 Reaktor in der MNT-Stufe, 2 Reaktoren in der DNT-Stufe, Abb. 2) zusammengestellt.

Der Vergleich der Nitrier- und Produktparameter für eine Nitrierung von Toluol zu DNT nach dem Standardverfahren mit insgesamt 5 Reaktoren mit dem verbesserten Verfahren mit nur 3 Reaktoren zeigt, dass ohne Änderung der Dosierparameter für Toluol, Salpetersäure und Schwefelsäure in den einzelnen Nitrierstufen ein MNT mit bis zu 5 % Toluol in der MNT-Stufe hergestellt werden und in der DNT-Stufe problemlos zu einem spezifikationsgerechten DNT umgesetzt werden kann, ohne dass im Vergleich zum Standardverfahren die für eine technische Nitrierung als optimal entwickelten allgemeinen Nitrierbedingungen geändert werden müssen.

Die Abfallsäuren auf der MNT- und der DNT-Stufe als Indikator für einen einwandfreien Ablauf der Nitrierung haben bei dem verbesserten Verfahren mit einer reduzierten Anzahl von Reaktoren die gleiche Zusammensetzung wie im Standardverfahren.

**Tabelle 1**

| | | **Beispiel 1 (Vergleich)** | **Beispiel 2 (Erfindungsgemäß)** | | | | **Beispiel 1 (Vergleich)** | **Beispiel 2** (**Erfindungsgemäß**) |
|---|---|---|---|---|---|---|---|---|
| **MNT-Stufe** | | | | | **DNT-Stufe** | | | |
| **Anzahl Reaktoren** | | 2 | 1 | | **Anzahl Reaktoren** | | 3 | 2 |
| **Nitriertemperatur MNT-Abfallsäure** | | 45° | 55° | | **Nitriertemperatur DNT-Abfallsäure** | | 60-70° | 65-80° |
| **Schwefelsäure** | **%** | 71.52 | 71.55 | | **Schwefelsäure** | **%** | 78.44 | 78.36 |
| **Salpetersäure** | **%** | 0.41 | 0.96 | | **Salpetersäure** | **%** | 1.20 | 1.19 |
| **Salpetrige Säure Produkt (MNT)** | **%** | 1.19 | 1.28 | | **Salpetrige Säure Produkt (DNT)** | **%** | 1.05 | 1.23 |
| **Toluol** | **%** | <0.15 | 3.5 | | **MNT** | **%** | n.n. | n.n. |
| **MNT** | **%** | 91.1 | 88.7 | | **TNT** | **%** | 0.01-0.02 | <0.01 |
| **DNT** | **%** | 8.8 | 7.8 | | **2,4 DNT** | **%** | 79.3 | 79.3 |
| | | | | | **2,6 DNT** | **%** | 20.7 | 20.7 |
| | | | | | **Andere Isomere** | **%** | 4.79 | 4.82 |

## Patentansprüche

1. Verfahren zur Herstellung von Dinitrotoluol im Gegenstrom, umfassend die Schritte
a) Umsetzung von Toluol mit Salpetersäure in Anwesenheit von Schwefelsäure zu Mononitrotoluol,
b) Trennung des Reaktionsprodukts aus Schritt a) in eine Mononitrotoluol enthaltende organische Phase und eine Schwefelsäure enthaltende wässrige Phase,
c) Umsetzung der Mononitrotoluol enthaltenden organischen Phase mit Salpetersäure in Anwesenheit von Schwefelsäure zu Dinitrotoluol,
d) Trennung des Reaktionsprodukts aus Schritt c) in eine Dinitrotoluol enthaltende organische Phase und eine Schwefelsäure enthaltende wässrige Phase,
**dadurch gekennzeichnet, dass** das Reaktionsprodukt aus Schritt a) einen Gehalt an Toluol von 3,5 - 8 Gew.-%, bezogen auf die organische Phase, und einen Gehalt an Salpetersäure von 0,1 bis 1,2 Gew.-%, bezogen auf die wässrige Phase, aufweist und die Phasentrennung in Schritt b) mittels dynamischer Separatoren durchgeführt wird.

2. Verfahren nach Anspruch 1, **dadurch gekennzeichnet, dass** das Reaktionsprodukt aus Schritt a) einen Gehalt an Toluol von 3,5 bis 5 Gew.-% bezogen auf das Gewicht der organischen Phase aus Schritt a), aufweist.

3. Verfahren nach Anspruch 1, **dadurch gekennzeichnet, dass** die Mononitrotoluol enthaltende organische Phase aus Schritt b) ohne weitere Aufarbeitung in Schritt c) überführt wird.

4. Verfahren nach Anspruch 1, **dadurch gekennzeichnet, dass** die Schwefelsäure enthaltenden wässrigen Phasen aus den Schritten b) und d), gegebenenfalls nach einer Aufarbeitung und Aufkonzentration, wieder in Schritt a) und c) eingesetzt werden.

5. Verfahren nach Anspruch 1, **dadurch gekennzeichnet, dass** als Reaktionsapparate für die Schritte a) und c) Rührkessel und/oder Strömungsreaktoren eingesetzt werden.

6. Verfahren nach Anspruch 1, **dadurch gekennzeichnet, dass** Schritt a) in nur einem Reaktionsapparat durchgeführt wird.

7. Verfahren nach Anspruch 1, **dadurch gekennzeichnet, dass** Schritt c) in maximal zwei in Reihe geschalteten Reaktionsapparaten durchgeführt wird.

8. Verfahren nach Anspruch 1, **dadurch gekennzeichnet, dass** Schritt a) bei einer Temperatur im Bereich zwischen 35 und 70°C durchgeführt wird.

9. Verfahren nach Anspruch 1, **dadurch gekennzeichnet, dass** Schritt c) bei einer Temperatur im Bereich zwischen 60 und 85°C durchgeführt wird.

10. Verfahren nach Anspruch 1, **dadurch gekennzeichnet, dass** das Mol-Verhältnis von Salpetersäure zu Toluol in Stufe a) im Bereich zwischen 0,95 und 1,12 liegt.

11. Verfahren nach Anspruch 1, **dadurch gekennzeichnet, dass** das Mol-Verhältnis von Salpetersäure zu Mononitrotoluol in Stufe c) im Bereich zwischen 1,03 und 1,10 liegt.

12. Verfahren nach Anspruch 1, **dadurch gekennzeichnet, dass** die Schwefelsäure enthaltende wässrige Phase aus Stufe b) zu einer Schwefelsäure mit einem mit einer Konzentration von 85 bis 96 % aufkonzentriert und in Stufe a) zurückgeführt wird.

13. Verfahren nach Anspruch 1, **dadurch gekennzeichnet, dass** die Schwefelsäure enthaltende wässrige Phase aus Stufe d) mit Salpetersäure versetzt und in Stufe a) zurückgeführt wird.

14. Verfahren nach Anspruch 1, **dadurch gekennzeichnet, dass** die zugeführte Salpetersäure in Stufe a) und Stufe c) eine Konzentration von 58 bis 100 % aufweist.

## Claims

1. A process for preparing dinitrotoluene in countercurrent, comprising the steps of
a) reacting toluene with nitric acid in the presence of sulfuric acid to give mononitrotoluene,
b) separating the reaction product from step a) into an organic phase comprising mononitrotoluene and an aqueous phase comprising sulfuric acid,
c) reacting the organic phase comprising mononitrotoluene with nitric acid in the presence of sulfuric acid to give dinitrotoluene,
d) separating the reaction product from step c) into an organic phase comprising dinitrotoluene and an aqueous phase comprising sulfuric acid,
wherein the reaction product from step a) has a content of toluene of 3.5-8% by weight, based on the organic phase, and a content of nitric acid of from 0.1 to 1.2% by weight, based on the aqueous phase, and the phase separation in step b) is carried out by means of dynamic separators.

2. The process according to claim 1, wherein the reaction product from step a) has a content of toluene of from 3.5 to 5% by weight based on the weight of the organic phase from step a).

3. The process according to claim 1, wherein the organic phase comprising mononitrotoluene from step b) is transferred to step c) without further workup.

4. The process according to claim 1, wherein the aqueous phases comprising sulfuric acid from steps b) and d), optionally after a workup and concentration, are reused in step a) and c).

5. The process according to claim 1, wherein the reaction apparatus used for steps a) and c) are stirred tanks and/or flow reactors.

6. The process according claim 1, wherein step a) is carried out in only one reaction apparatus.

7. The process according to claim 1, wherein step c) is carried out in a maximum of two reaction apparatus connected in series.

8. The process according to claim 1, wherein step a) is carried out at a temperature in the range between 35 and 70°C.

9. The process according to claim 1, wherein step c) is carried out at a temperature in the range between 60 and 85°C.

10. The process according to claim 1, wherein the molar ratio of nitric acid to toluene in stage a) is in the range between 0.95 and 1.12.

11. The process according to claim 1, wherein the molar ratio of nitric acid to mononitrotoluene in stage c) is in the range between 1.03 and 1.10.

12. The process according to claim 1, wherein the aqueous phase comprising sulfuric acid from stage b) is concentrated to give sulfuric acid having a concentration of from 85 to 96% and recycled in stage a).

13. The process according to claim 1, wherein the aqueous phase comprising sulfuric acid from stage d) is admixed with nitric acid and recycled into stage a).

14. The process according to claim 1, wherein the nitric acid supplied in stage a) and stage c) has a concentration of from 58 to 100%.

## Revendications

1. Procédé pour la production de dinitrotoluène à contre-courant, comprenant les étapes
a) mise en réaction de toluène avec de l'acide nitrique en présence d'acide sulfurique, pour l'obtention de mononitrotoluène,
b) fractionnement du produit de réaction provenant de l'étape a) en une phase organique contenant du mononitrotoluène et une phase aqueuse contenant de l'acide sulfurique,
c) mise en réaction de la phase organique contenant du mononitrotoluène avec de l'acide nitrique en présence d'acide sulfurique, pour l'obtention de dinitrotoluène,
d) fractionnement du produit de réaction provenant de l'étape c) en une phase organique contenant du dinitrotoluène et une phase aqueuse contenant de l'acide sulfurique,
**caractérisé en ce que** le produit de réaction provenant de l'étape a) présente une teneur en toluène de 3,5-8 % en poids, par rapport à la phase organique, et une teneur en acide nitrique de 0,1 à 1,2 % en poids ; par rapport à la phase aqueuse, et la séparation de phases dans l'étape b) est effectuée au moyen de séparateurs dynamiques.

2. Procédé selon la revendication 1, **caractérisé en ce que** le produit de réaction provenant de l'étape a) présente une teneur en toluène de 3,5 à 5 % en poids par rapport au poids de la phase organique provenant de l'étape a).

3. Procédé selon la revendication 1, **caractérisé en ce que** la phase organique contenant du mononitrotoluène, provenant de l'étape b), est transférée dans l'étape c) sans être traitée davantage.

4. Procédé selon la revendication 1, **caractérisé en ce que** les phases aqueuses contenant de l'acide sulfurique, provenant des étapes b) et d), sont réutilisées dans l'étape a) et l'étape c) éventuellement après un traitement final et une concentration.

5. Procédé selon la revendication 1, **caractérisé en ce qu'**on utilise comme appareils de réaction pour les étapes a) et c) des cuves à agitation et/ou des réacteurs à circulation.

6. Procédé selon la revendication 1, **caractérisé en ce que** l'étape a) est effectuée dans un seul appareil de réaction.

7. Procédé selon la revendication 1, **caractérisé en ce que** l'étape c) est effectuée dans au maximum deux appareils de réaction raccordés en série.

8. Procédé selon la revendication 1, **caractérisé en ce que** l'étape a) est effectuée à une température dans la plage comprise entre 35 et 70°C.

9. Procédé selon la revendication 1, **caractérisé en ce que** l'étape c) est effectuée à une température dans la plage comprise entre 60 et 85°C.

10. Procédé selon la revendication 1, **caractérisé en ce que** le rapport molaire de l'acide nitrique au toluène dans l'étape a) se situe dans la plage comprise entre 0,95 et 1,12.

11. Procédé selon la revendication 1, **caractérisé en ce que** le rapport molaire de l'acide nitrique au mononitrotoluène dans l'étape c) se situe dans la plage comprise entre 1,03 et 1,10.

12. Procédé selon la revendication 1, **caractérisé en ce que** la phase aqueuse contenant de l'acide sulfurique, provenant de l'étape b), est concentrée en un acide sulfurique à une concentration de 85 à 96 % et renvoyée dans l'étape a).

13. Procédé selon la revendication 1, **caractérisé en ce qu'**on ajoute de l'acide nitrique à la phase aqueuse contenant de l'acide sulfurique, provenant de l'étape d), et on la renvoie dans l'étape a).

14. Procédé selon la revendication 1, **caractérisé en ce que** l'acide nitrique introduit dans l'étape a) et dans l'étape c) présente une concentration de 58 à 100 %.
